**Europäisches Patentamt**

**European Patent Office**

**Office européen des brevets**

(19)

(11) Numéro de publication: **0 005 106**
**A2**

(12)

# DEMANDE DE BREVET EUROPEEN

(21) Numéro de dépôt: **79400238.6**

(51) Int. Cl.²: **A 61 N 1/04**

(22) Date de dépôt: **12.04.79**

(30) Priorité: **19.04.78 FR 7811512**

(43) Date de publication de la demande:
**31.10.79 Bulletin 79/22**

(84) Etats Contractants Désignés:
**BE CH DE GB IT LU NL SE**

(71) Demandeur: **SYNTHELABO**
**1, avenue de Villars**
**F-75341 Paris Cedex 07(FR)**

(72) Inventeur: **Balat, Roger**
**16 rue Nansouty**
**F-75014Paris(FR)**

(72) Inventeur: **Illes, Joseph Antonin**
**8 rue Lyautey**
**F-92340 Bourg-La-Reine(FR)**

(72) Inventeur: **Jacquemart, Jean-François Victor**
**89 rue Pelleport**
**F-75020 Paris(FR)**

(72) Inventeur: **Sarda, Christian**
**Le Petit Nassaudre**
**F-27470 Serquigny(FR)**

(74) Mandataire: **CASANOVA, André et al,**
**Cabinet Casanova et Akerman 23 Boulevard de**
**Strasbourg**
**F-75010 Paris(FR)**

(54) Sonde de stimulation cardiaque endocavitaire.

(57) L'invention concerne une sonde de stimulation cardiaque endocavitaire.

Cette sonde comprend par exemple une électrode tubulaire 1, reliée au stimulateur cardiaque par un conducteur souple, tubulaire, isolé 2; un harpon 5a, en une matière solide, biocompatible et résorbable en une durée de l'ordre de 3 semaines à 3 mois, est monté coulissant dans l'électrode tubulaire 1, de manière à pouvoir en être extrait, en vue de l'implantation dans la paroi endocavitaire, par une poussée transmise par exemple par un fil 8, traversant le conducteur tubulaire 2.

Cette sonde a l'avantage d'être immobilisée d'abord par le harpon, puis, après sa résorption, par la formation naturelle de fibrose autour de l'électrode.

Fig.1

EP 0 005 106 A2

1

Sonde de stimulation cardiaque endocavitaire.
(Invention de Roger BALAT, Joseph, Antonin ILLES,
Jean-François, Victor JACQUEMART et Christian SARDA).
----------------------

La présente invention concerne une sonde de
stimulation cardiaque endocavitaire.

On connaît déjà des sondes de ce genre, qui
comportent chacune une électrode et un conducteur souple,
isolé extérieurement, pour relier ladite électrode à un
stimulateur cardiaque. L'extrémité d'une telle sonde est
introduite, par voie veineuse, dans une cavité cardiaque,
de manière à amener son électrode terminale au contact
de la zone endocavitaire à stimuler. C'est seulement
après une période qui s'étend de trois semaines à trois
mois environ que la formation de fibrose autour de l'électrode garantit l'assujettissement de cette dernière
au contact de la zone endocavitaire à stimuler ; bien
entendu, pendant la période intermédiaire, il existe un
risque important, évalué de 4 à 15 %, que l'électrode de
la sonde ne se déplace par rapport à la zone précise à
stimuler, en particulier sous l'effet des contractions
du myocarde.

C'est pourquoi on a déjà réalisé des sondes
de stimulation cardiaque endocavitaire, comportant des
moyens pour assujettir de façon permanente leur électrode
à la zone endocavitaire à stimuler. Les électrodes de
ces sondes perfectionnées comportent à leur extrémité des organes d'ancrage, tels que des barbes métalliques, en nombre variable, des hélices, simples ou doubles, en fil métallique... etc. Certains de ces organes d'ancrage, en particulier ceux à barbes métalliques, ne sont pas parfaitement fiables, dans la mesure où les contractions du myocarde peuvent, dans certains cas, produire leur désancrage. D'autre part, l'explantation de la sonde, parfois nécessaire, nécessite le désancrage de son électrode, ce qui risque d'endommager localement la paroi endocavitaire. Dans le cas par contre où l'électrode d'une telle sonde reste implantée dans la paroi endocavitaire pendant plusieurs années, des problèmes importants peuvent résulter de l'incompatibilité entre l'organe d'ancrage métallique de ladite électrode, d'une part, et la paroi cardiaque d'autre part. Enfin, même après la formation de fibrose autour de l'électrode d'une telle sonde, munie d'un organe d'ancrage, on a pu constater des déplacements de ladite électrode par rapport à la zone endocavitaire à stimuler ; ces phénomènes, extrêmement gênants, seraient dus aux actions mécaniques auxquelles le ou les organes d'ancrage sont soumis en raison des contractions du myocarde. Parmi ces sondes connues, certaines sont en outre pourvues de moyens pour neutraliser l'organe d'ancrage tant que l'électrode n'est pas parvenue au contact de la zone endocavitaire à stimuler.

La demande de brevet français N° 74.03439, déposée par Siemens A.G. le 1er février 1974, décrit une sonde de stimulation cardiaque endocavitaire, dont l'organe d'immobilisation de l'électrode est constitué par un ballonnet gonflable au moyen d'un fluide, amené par un tube, dont est solidaire le conducteur souple reliant l'électrode au stimulateur cardiaque. Un tel moyen d'immobilisation de l'électrode est peu fiable.

La demande de brevet allemand N° 2 453 840 déposée par M. Lampadius le 13 novembre 1974 décrit une sonde de stimulation cardiaque endocavitaire, dont l'électrode peut être collée à la zone endocavitaire à stimuler

0005106

par un adhésif, contenu dans un petit réservoir adapté à l'électrode. Cet adhésif peut être une substance auto-polymérisante, compatible avec le tissu cardiaque, et susceptible de durcir en combinaison avec le liquide dudit tissu, puis d'être résorbée par le tissu cardiaque lui-même. Une telle fixation de l'électrode de stimulation, par simple collage, est également peu fiable.

La sonde de stimulation cardiaque endocavitaire selon la présente invention comporte également une électrode, munie d'un organe d'ancrage à la paroi endocavitaire, des moyens pour neutraliser l'organe d'ancrage tant que l'électrode n'est pas parvenue au contact de la zone endocavitaire à stimuler, et un conducteur souple, isolé extérieurement, pour relier ladite électrode à un stimulateur cardiaque, mais elle ne présente pas les inconvénients, précédemment mentionnés des sondes de ce type connu.

La sonde de stimulation cardiaque endocavitaire selon la présente invention est caractérisée en ce que l'organe d'ancrage de son électrode à la paroi endocavitaire est constitué, au moins dans sa partie active, en une matière solide, biocompatible et résorbable dans une période de l'ordre de trois semaines à trois mois.

L'organe d'ancrage de la sonde selon la présente invention, neutralisé pendant l'introduction de ladite sonde par voie artérielle, n'entre en activité que lorsque l'électrode de la sonde est parvenue au contact de la paroi endocavitaire, précisément dans la zone à stimuler ; il pénètre alors dans cette zone de la paroi endocavitaire, à laquelle il assujettit d'une façon parfaitement fiable ladite électrode pendant toute la période, de l'ordre de trois semaines à trois mois, qui est nécessaire pour la formation naturelle de fibrose ; à l'issue de cette période, l'organe d'ancrage,ou tout au moins sa partie active, s'est complètement résorbé , mais l'électrode de la sonde demeure assujettie à la zone endocavitaire à stimuler par la fibrose formée.
On évite donc ainsi que l'électrode de la sonde ne se
déplace par rapport à la zone endocavitaire à stimuler,
pendant la période initiale, de l'ordre de trois semaines
à trois mois, grâce à l'organe d'ancrage, puis, après
la résorption de ce dernier, grâce à la fibrose formée,
qui assure la fixation définitive de l'électrode. Cette
fixation définitive de l'électrode n'est cependant pas
obtenue par un organe d'ancrage, introduit en permanence
dans la paroi endocavitaire. Il en résulte les avantages
suivants : les contractions du myocarde n'exerçant pas
des actions mécaniques directes sur l'électrode de la
sonde selon la présente invention, tout au moins après
résorption de son organe d'ancrage, il n'y a plus aucun
risque de déplacement de l'électrode par rapport à la
zone à stimuler ; d'autre part, l'explantation de l'électrode ne nécessite plus l'extraction d'un organe d'ancrage hors de la paroi endocavitaire, ce qui élimine les
risques élevés correspondants.

Dans une forme de réalisation préférée de la
sonde de stimulation cardiaque endocavitaire selon la
présente invention, son électrode comporte une cavité,
qui présente une ouverture à l'extrémité de la sonde, et
dans laquelle est logé l'organe d'ancrage, et des moyens
sont prévus pour extraire seulement la partie active
dudit organe d'ancrage par l'ouverture de ladite cavité,
lorsque l'électrode est parvenue au contact de la zone
endocavitaire à stimuler. Par exemple, l'électrode a une
forme tubulaire, et la partie active de l'organe d'ancrage est portée par une pièce, mobile dans ladite électrode tubulaire.

L'extraction de la partie active de l'organe
d'ancrage hors de l'électrode tubulaire peut être obtenue par différents moyens, entrant tous dans le cadre
de la présente invention :

Dans une première réalisation, la pièce portant la partie active de l'organe d'ancrage, ou bien un

5

piston monté coulissant dans l'électrode tubulaire, derrière ledit organe d'ancrage, est constitué en un matériau sensible aux actions à distance, par exemple de nature magnétique, électrique ou électro-magnétique.

L'extraction de la partie active de l'organe d'ancrage et son implantation dans la paroi endocavitaire peuvent alors être commandées par des actions à distance, qui peuvent être produites, de façon connue en soi, à l'extérieur du corps du patient, au niveau de son thorax.

Dans une autre forme de réalisation, le conducteur souple, isolé extérieurement, a la forme d'un tube, par exemple une tresse métallique, pour la transmission, depuis l'extrémité extra-cardiaque de la sonde, d'une poussée d'extraction à l'organe d'ancrage, ou à un piston monté coulissant dans l'électrode tubulaire, derrière ledit organe d'ancrage ; l'étanchéité de l'électrode tubulaire est alors assurée par une conformation appropriée de la pièce portant la partie active de l'organe d'ancrage et/ou dudit piston. Quant à l'agent de transmission de la poussée d'extraction, il peut être un liquide, notamment un silicone liquide, remplissant le conducteur tubulaire. Ce peut être aussi un fil souple, monté coulissant dans le conducteur tubulaire.

Cette dernière forme de réalisation de la sonde selon la présente invention est particulièrement avantageuse dans la mesure où elle permet l'explantation de l'électrode même pendant la période initiale, antérieure à la formation de fibrose, et précédant la résorption de l'organe d'ancrage implanté dans la paroi endocavitaire. Pour cela, en effet, il suffit de réintroduire dans le conducteur tubulaire le fil souple qui, lors de l'implantation, a servi à l'extraction de l'organe d'ancrage hors de l'électrode tubulaire, et à son implantation dans la paroi endocavitaire, et d'exercer sur l'organe d'ancrage, directement ou par l'intermédiaire du piston coulissant, en utilisant ce fil souple ou, si besoin est, un fil plus rigide, une poussée suffisante pour extraire à son tour de l'électrode tubulaire la pièce portant la partie active de l'organe d'ancrage; lorsque ce dernier est complètement sorti de l'électrode tubulaire il est possible de retirer la sonde sans aucun risque d'endommagement de la paroi endocavitaire, l'organe d'ancrage qui y reste inséré étant ultérieurement résorbé.

A titre d'exemple, on a décrit ci - dessous et illustré schématiquement au dessin annexé, une forme de réalisation de la sonde de stimulation cardiaque endocavitaire selon la présente invention.

La figure 1 montre l'extrémité de cette sonde, qui porte l'électrode tubulaire, en coupe par un plan axial de cette électrode.

La figure 2 illustre l'implantation de la sonde de la figure 1 dans une paroi endocavitaire.

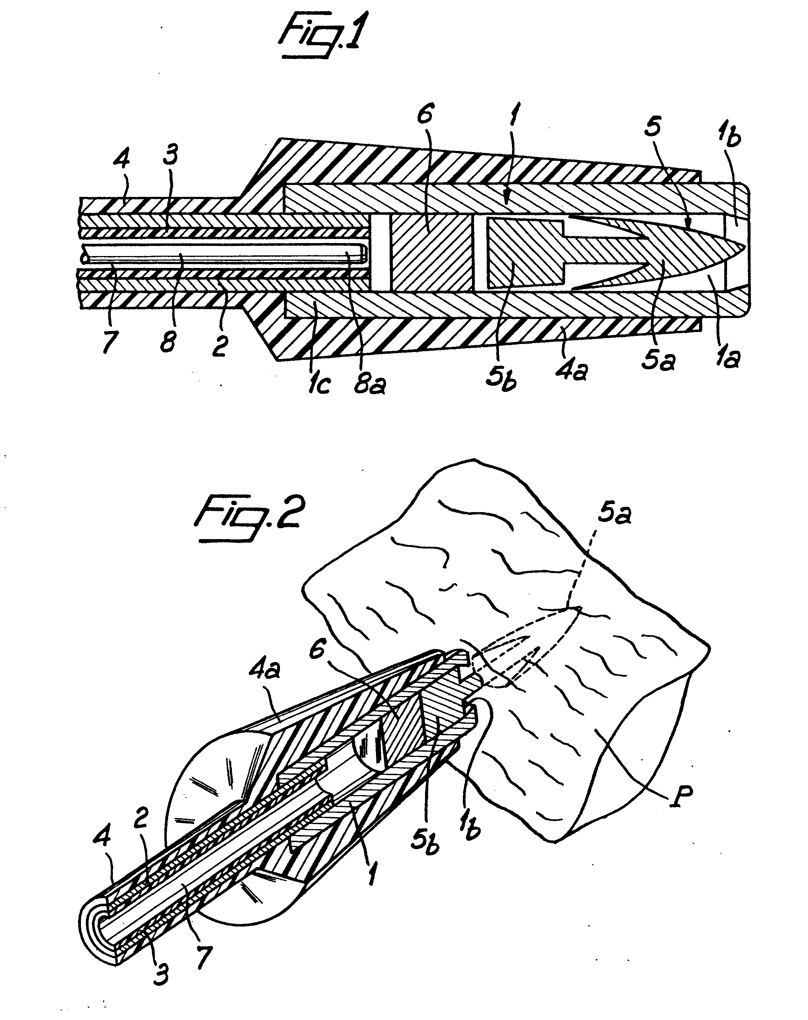

Sur la figure 1, 1 désigne une électrode tubulaire, par exemple en platine, dont l'évidement intérieur, cylindrique, la, débouche à l'extrémité de la sonde, par une ouverture lb, présentant une légère conicité vers l'extérieur. Dans l'extrémité intérieure, lc, de l'électrode tubulaire 1 est fixée, par tous moyens appropriés, par exemple par soudage, l'extrémité d'un conducteur souple, 2, en forme de tube, par exemple une tresse métallique en fils d'acier inoxydable. Ce conducteur souple, en forme de tube, dont on a seulement représenté sur la figure 1 la partie voisine de l'électrode 1, s'étend sur toute la longueur de la sonde, qui doit être suffisante pour permettre son introduction, par voie artérielle, dans une cavité cardiaque, de façon connue en soi. Le conducteur tubulaire 2 est isolé électriquement, intérieurement, par une gaine de polyéthylène 3, qui s'étend sur toute sa longueur, et, extérieurement, par exemple par une couche 4 de silicone de qualité médicale, qui est surmoulée sur ledit conducteur tubulaire 2, ainsi que, en 4a, par-dessus l'électrode tubulaire 1, de manière cependant à dégager son extrémité extérieure

0005106

et l'ouverture correspondante 1b.

L'équipement de la sonde, avant son utilisation, telle qu'elle est illustrée sur la figure 1, comporte en outre les organes suivants : dans la cavité intérieure cylindrique, 1a, de l'électrode tubulaire 1 est logé un organe d'ancrage 5 ; celui-ci, dans la forme de réalisation considérée, comprend une partie active 5a, qui a par exemple la forme d'un harpon, et qui est portée par une pièce 5b ; celle-ci peut être par exemple tronconique, et dimensionnée de manière qu'au moins sa grande base, située vers l'intérieur de l'électrode tubulaire 1, ait un diamètre un peu supérieur au diamètre minimum de l'embouchure tronconique 1b. Selon la présente invention, les deux parties, 5a et 5b, de l'organe d'ancrage 5, ou tout au moins sa partie active 5a, sont constituées en une matière solide, biocompatible et résorbable dans une période de l'ordre de trois semaines à trois mois ; de telles matières sont connues, et utilisées en particulier pour la réalisation de sutures chirurgicales ; il peut s'agir en particulier de catgut, c'est-à-dire de boyaux animaux, torsadés, et par exemple dopés avec une matière telle que le chrome, pour en accroître la durée de résorption ; on peut utiliser également des matières collagènes ou des matières synthétiques, qui sont biocompatibles et résorbables naturellement dans les délais indiqués, par exemple un polymère de l'acide polyglycolique, ou un copolymère d'acide polyglycolique (90 %) et d'acide lactique (10 %). Derrière la pièce 5b de l'organe d'ancrage 5, vers l'intérieur de l'électrode tubulaire 1, est monté coulissant dans cette dernière, un piston 6, par exemple en platine ; la surface latérale de ce piston 6, de même que la paroi interne de l'électrode tubulaire 1 présentent un fini approprié pour assurer l'étanchéité de la sonde vers l'intérieur du conducteur tubulaire 2, afin d'éviter toute pénétration, dans le canal intérieur 7, de liquides biologiques, susceptibles de remonter jusqu'à l'extrémité dudit conducteur qui est reliée au stimulateur cardiaque (non représenté) ; une telle remontée d'un liquide biologique, plus ou moins conducteur, risquerait en effet de court-circuiter la sortie du stimulateur cardiaque et d'empêcher son fonctionnement correct. Enfin, un fil d'acier 8 est monté librement coulissant dans le canal intérieur 7 du conducteur tubulaire 2, dont il est isolé électriquement par la gaine de polyéthylène 3 ; il s'agit de préférence d'un fil d'acier inoxydable.

Pour indiquer l'ordre de grandeur de la tête de la sonde illustrée, à grande échelle, sur la figure 1, on précisera que, par exemple, l'électrode tubulaire, 1,présente de préférence un diamètre extérieur de l'ordre de 2,5 mm et un diamètre intérieur de l'ordre de 1,5 mm.

La tête de la sonde illustrée sur la figure 1 est introduite par voie veineuse, de façon connue en soi, jusque dans la cavité cardiaque où se trouve la zone à stimuler. Lorsque l'embouchure 1b de l'électrode tubulaire 1 est parvenue au contact de la paroi endocavitaire P, comme illustré sur la figure 2, l'opérateur pousse l'extrémité extra-cardiaque du fil métallique 8 vers l'intérieur, c'est-à-dire en direction de l'électrode 1, si bien que l'extrémité 8a du fil 8 pousse le piston 6 en direction de la partie 5b de l'organe d'ancrage 5 ; un déplacement de quelques millimètres du fil 8 dans la gaine souple, mais fixe, 3-2-4, suffit pour produire l'extraction, hors de l'électrode tubulaire 1, de la partie active, 5a, de l'organe d'ancrage 5, par l'action du piston d'étanchéité 6 ; la partie active 5a de l'organe d'ancrage 5 traverse alors l'endocarde de la paroi endocavitaire P, puis vient s'enficher entièrement dans le myocarde à la façon d'un harpon, tandis que la partie 5b de l'organe d'ancrage 5 est retenue dans l'extrémité de l'électrode tubulaire 1, grâce à la conicité de l'embouchure 1b de cette dernière. Lorsque l'implantation de l'organe d'ancrage 5 a eu lieu, l'opérateur retire le fil métallique 8 de la sonde, dont l'extrémité, extracardiaque, du conducteur tubulaire 2 peut être alors reliée
à la sortie du stimulateur cardiaque, de façon connue en
soi.

Comme on l'a indiqué précédemment, la partie active 5a de l'organe d'ancrage 5 est complètement
résorbée lorsqu'il s'est développé, autour de l'extrémité
libre de l'électrode tubulaire 1, suffisamment de fibrose
pour assujettir de façon permanente l'extrémité de ladite
électrode 1 à la paroi endocavitaire P. Même avant cette
résorption complète de la partie active, 5a, de l'organe
d'ancrage 5, la sonde selon la présente invention peut
cependant être explantée en réintroduisant dans le canal
intérieur 7 un fil métallique tel que 8, ou un fil de
diamètre un peu plus grand, et présentant par suite une
plus grande rigidité, permettant d'exercer sur la partie
5b de l'organe d'ancrage 5, par l'intermédiaire du piston
6, une poussée suffisante pour faire sortir complètement
ladite partie 5b de l'électrode tubulaire 1, à travers
son ouverture conique 1b ; ayant ainsi complètement séparé l'organe d'ancrage 5 de l'électrode 1, il devient possible de retirer l'ensemble de la sonde.

La sonde selon la présente invention est
susceptible de nombreuses formes de réalisation, qui
diffèrent de celle précédemment décrite, mais qui entrent
toutes dans le cadre de l'invention : si le fil souple 8
est en un matériau électriquement isolant, ou s'il est
revêtu d'une gaine d'un tel matériau, on peut supprimer
la gaine de polyéthylène 3, destinée à assurer l'isolation intérieure du conducteur tubulaire 2. La poussée
d'extraction peut être transmise,par l'intermédiaire du
piston 6, à l'organe d'ancrage 5, non pas par un fil
solide 8, mais par un liquide, notamment un silicone liquide, remplissant le canal intérieur 7 du conducteur
tubulaire 2 ; dans ce cas, la poussée d'extraction peut
être produite au moyen d'une pompe, notamment à piston,
dont le refoulement est relié à l'extrémité extracardia-

0005106

que du canal intérieur 7 du conducteur tubulaire 2. La partie 5b de l'organe d'ancrage 5 qui porte sa partie active 5a peut aussi être conformée de manière à assurer l'étanchéité de l'électrode tubulaire 1 vers l'intérieur du conducteur tubulaire 2 ; dans ce cas, le piston 6 peut être éventuellement supprimé. Cette partie, 5b, de l'organe d'ancrage 5, n'est pas obligatoirement constituée par un matériau biocompatible ou tout au moins résorbable, bien que cela soit préférable en cas d'explantation de l'électrode. Dans une forme de réalisation particulière, la partie 5b de l'organe d'ancrage, ou bien le piston 6, monté coulissant dans l'électrode tubulaire 1, derrière l'organe d'ancrage 5, peut être constitué en un matériau sensible aux actions à distance, par exemple de nature magnétique, électrique ou électromagnétique ; par exemple, la partie 5b de l'organe d'ancrage 5 et/ou le piston 6 peut être constitué, au moins partiellement, en un matériau magnétique, sensible à l'action d'un électro-aimant, dont le déplacement, à l'extérieur du patient, au niveau de son thorax, peut alors permettre l'extraction de l'organe d'ancrage 5, ou tout au moins de sa partie active 5a, hors de l'électrode tubulaire 1. Dans ce cas, le fil 8 peut être supprimé, de même éventuellement que le piston 6, et le conducteur 2 n'est plus nécessairement de forme tubulaire.

La forme tubulaire de l'électrode 1 et la forme cylindrique de sa cavité intérieure, et par suite des éléments 5b et 6 qui y sont placés, sont également matières à option ; le fonctionnement, précédemment décrit à l'aide de la figure 2, peut être obtenu avec une électrode de forme quelconque, pourvu qu'elle comporte une cavité, qui présente une ouverture à l'extrémité de la sonde, et dans laquelle est logé l'organe d'ancrage.

La présente invention s'étend également à la substitution, aux organes d'ancrage, généralement métalliques, des sondes de stimulation cardiaque endocavitaire, antérieurement connues, d'organes d'ancrage constitués au moins partiellement en une matière solide,
biocompatible et résorbable dans une période appropriée.

1

0005106

REVENDICATIONS DE BREVET

1.- Sonde de stimulation cardiaque endocavitaire, comportant une électrode, munie d'un organe
d'ancrage à la paroi endocavitaire, des moyens pour neutraliser l'organe d'ancrage tant que l'électrode n'est
pas parvenue au contact de la zone endocavitaire à stimuler, et un conducteur souple, isolé extérieurement, pour
relier ladite électrode à un stimulateur cardiaque, cette
sonde étant caractérisée en ce que l'organe d'ancrage de
l'électrode à la paroi endocavitaire est constitué, au
moins dans sa partie active, en une matière solide, biocompatible et résorbable dans une période de l'ordre de
trois semaines à trois mois.

2.- Sonde selon la revendication 1, caractérisée en ce que l'électrode comporte une cavité qui
présente une ouverture à l'extrémité de la sonde, et
dans laquelle est logé l'organe d'ancrage, et que des
moyens sont prévus pour extraire seulement la partie active dudit organe d'ancrage par l'ouverture de ladite
cavité, lorsque l'électrode est parvenue au contact de
la zone endocavitaire à stimuler.

3.- Sonde selon la revendication 2, caractérisée en ce que l'électrode a une forme tubulaire, et
que la partie active de l'organe d'ancrage est portée par
une pièce, mobile dans ladite électrode tubulaire.

4.- Sonde selon la revendication 3, caractérisée en ce que la pièce portant la partie active de
l'organe d'ancrage, ou bien un piston monté coulissant
dans l'électrode tubulaire, derrière ledit organe d'ancrage, est constitué en un matériau sensible aux actions
à distance, par exemple de nature magnétique, électrique
ou électromagnétique.

5.- Sonde selon la revendication 3, caractérisée en ce que le conducteur souple, isolé extérieurement, a la forme d'un tube, par exemple une tresse métallique, pour la transmission, depuis l'extrémité extra-
cardiaque de la sonde, d'une poussée d'extraction, à l'organe d'ancrage, ou à un piston monté coulissant dans l'électrode tubulaire, derrière ledit organe d'ancrage, et que l'étanchéité de l'électrode tubulaire est assurée par une conformation appropriée de la pièce portant la partie active de l'organe d'ancrage et/ou dudit piston.

6.- Sonde selon la revendication 5, caractérisée en ce que l'agent de transmission de la poussée d'extraction est un liquide, notamment un silicone liquide, remplissant le conducteur tubulaire.

7.- Sonde selon la revendication 5, caractérisée en ce que l'agent de transmission de la poussée d'extraction est un fil souple, monté coulissant dans le conducteur tubulaire.

8.- Sonde selon l'une quelconque des revendications 6 et 7, caractérisée en ce que, dans le cas où l'agent de transmission est électriquement conducteur, par exemple un fil métallique nu, le conducteur tubulaire est aussi isolé intérieurement.

*Fig.1*

*Fig.2*